(19) **Europäisches Patentamt / European Patent Office / Office européen des brevets**

(11) **EP 4 067 896 B1**

(12) **EUROPEAN PATENT SPECIFICATION**

(45) Date of publication and mention
of the grant of the patent:
**25.12.2024 Bulletin 2024/52**

(21) Application number: **22162231.9**

(22) Date of filing: **15.03.2022**

(51) International Patent Classification (IPC):
**G01N 30/06** (2006.01)     **G01N 30/88** (2006.01)
**A61M 5/178** (2006.01)     **G01N 1/28** (2006.01)

(52) Cooperative Patent Classification (CPC):
**G01N 30/88; A61M 5/349; G01N 1/286;**
**G01N 30/06;** G01N 2030/062; G01N 2030/067;
G01N 2030/884; G01N 2030/8886

(54) **METHOD FOR DETERMINING RESIDUAL ALDEHYDES RELEASABLE FROM A SYRINGE**

VERFAHREN ZUR BESTIMMUNG DER AUS EINER SPRITZE FREISETZBAREN RESTALDEHYDE

PROCÉDÉ DE DÉTERMINATION DES ALDÉHYDES RÉSIDUELS LIBÉRABLES D'UNE SERINGUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2021 IT 202100007835**

(43) Date of publication of application:
**05.10.2022 Bulletin 2022/40**

(73) Proprietor: **Nuova Ompi S.r.l.
35017 Piombino Dese (PD) (IT)**

(72) Inventors:
- **BESSEGATO, Nicola**
**35017 Piombino Dese (PD) (IT)**
- **CAMPESATO, Lara**
**35017 Piombino Dese (PD) (IT)**
- **SFULCINI, Alberto**
**35017 Piombino Dese (PD) (IT)**

(74) Representative: **Long, Giorgio
Jacobacci & Partners S.p.A.
Piazza Mario Saggin, 2
35131 Padova (IT)**

(56) References cited:
**CN-A- 102 411 034**

- **STAFIEJ A ET AL: "Screening and optimization of derivatization in heating block for the determination of aliphatic aldehydes by HPLC",** JOURNAL OF BIOCHEMICAL AND BIOPHYSICAL METHODS, AMSTERDAM, NL, vol. 69, no. 1-2, 30 November 2006 (2006-11-30), pages 15 - 24, XP024996792, ISSN: 0165-022X, [retrieved on 20061130], DOI: 10.1016/J.JBBM.2006.02.009
- **DE M OCHS S ET AL: "Optimization and comparison of HPLC and RRLC conditions for the analysis of carbonyl-DNPH derivatives",** TALANTA, ELSEVIER, AMSTERDAM, NL, vol. 81, no. 1-2, 15 April 2010 (2010-04-15), pages 521 - 529, XP026923841, ISSN: 0039-9140, [retrieved on 20100104], DOI: 10.1016/J.TALANTA.2009.12.036
- **SIEW ADELINE: "DRUG-DELIVERY SYSTEMS FOR BIOPHARMACEUTICALS", vol. 28, no. 8, 1 August 2015 (2015-08-01), pages 1 - 53, XP055872335, Retrieved from the Internet <URL:http://images2.advanstar.com/PixelMags/biopharm/pdf/2015-08.pdf#page=15> [retrieved on 20211214]**
- **UNKNOWN: "EXTRACTABLES & LEACHABLES CHEMICAL CHARACTERIZATION", 1 January 2017 (2017-01-01), pages 1 - 1, XP055872348, Retrieved from the Internet <URL:https://www.sgsgroup.de/-/media/local/germany/documents/brochures/lss/sgs_if_extractables_leachables_en.pdf> [retrieved on 20211213]**
- **"Introduction to Modern Chromatography", 9 December 2009, JOHN WILEY & SONS, article SNYDER LLOYD R. ET AL: "11.4.1.1 External Standardization", pages: 1 - 912, XP055872654**

EP 4 067 896 B1

**Description**

Technical field of the invention

The present invention relates to a method for extracting aldehydes from the adhesive used for gluing needles to syringes, and to a method for determining the aldehydes releasable from said syringes, before or after such a treatment.

Background art

CN 102 411 034 A relates to detecting residual aldehydes releasable from an adhesive or glue.

The development of protein-based medicaments poses major problems of contamination due to the interaction with the primary containers (e.g., syringes) in which they are placed for storage and subsequent administration. Such problems are described for example in paragraphs [0001-0007] of US20160251261A1 and in the article by Denfeng Liu et al., "Interactions between Therapeutic Proteins and Acrylic Acid Leachable," PDA J. Pharm. Sci. And Tech. 2012, 66, 12-19.

A particular class of substances which react negatively with such drugs, and which can be released from the primary container, are aldehydes, in particular formaldehyde and acrolein (but also acetaldehyde and methacrolein), which can be present in the syringe cone due to the adhesive used to attach the needle to the syringe, and released therefrom, in particular due to the interaction with some drugs.

Various methods of decreasing the release of substances from the glues used in syringes are known.

For example, it is known to appropriately vary the emission parameters (wavelength, type of light source, time profile and radiation intensity) used during the glue curing step.

However, such methods tend not to be specific, i.e., they have no particular effect with respect to the reduction of aldehydes, and moreover, when it comes to the reduction of such extractable classes to very low levels, such methods are not sufficiently stable.

It is also known to use autoclave treatments on primary containers, for the purpose of the sterilization thereof. Such a treatment is described in EP3496765A2, for example. However, such a process is generally not suitable for syringes as it can weaken the gluing seal of the needle or damage any rubber components (such as the piston or the needle shield cap) already pre-assembled on the syringe. Accordingly, EtO gas sterilization is preferred for this type of component.

In order to keep the syringe production process under control, it is also important to develop an aldehyde detection method which is capable of determining the presence thereof irrespective of the drug later introduced therein. Such a method must be highly precise and sensitive. It is known to use liquid chromatography as a detection method, but the results obtained depend on many factors, including the substance used for the so-called aldehyde derivatization.

In fact, aldehydes are not detectable per se at low concentrations (ng/mL) if they are not first bound to a substance which allows the discrimination thereof, forming a chromophore product (hydrazone) therewith which is determinable with a simple UV-Vis detector; such a chemical process is referred to as derivatization and allows determining the aldehydes at picomole levels with a significant increase in the detectability limits.

The choice of the derivatization agent also affects the sensitivity of the method; for example, some substances do not allow detecting very small amounts of aldehydes (of the order of ng/ml), because the derivatization yields are insufficient for producing an amount of product the signal of which can be determinable even with the most modern analytical techniques.

An example of an unsuitable binder is pentafluorobenzyl hydroxylamine (PFBHA). Although the reactivity of this molecule with carbonyl groups to generate oximes is known, the development of the reaction is analyte-specific. The determination of an aldehyde reacting with PFBHA is generally possible by a mass spectrometry detector, but the derivatization reaction in this case is very sensitive to the following variables: reaction time, temperature, pH, and reagent amount for each specific aldehyde. The effect of each variable is specific to each aldehyde and makes the overall efficiency of the reaction favorable for some aldehydes but simultaneously unfavorable for others. Therefore, using this derivatization reagent does not allow simultaneously determining the four aldehydes under investigation at ng/mL concentration levels.

Furthermore, it has been found that when the tests are performed by filling the syringe with a sample liquid, such as water, the amount of aldehydes detected is also affected by other factors not dependent on the amount and quality of adhesive present in the cone, such as aldehydes present in the plunger or in the water itself.

Based on the above, it is therefore necessary to develop a syringe production method which minimizes the release of aldehydes, without causing a worsening in the release of other substances, and which is independent of the type of drug with which the syringe is filled.

There is also a need to develop an analytical method for the determination of residual aldehydes releasable by a syringe, in particular a syringe subjected to a method for extracting aldehydes from the glue.

## Summary of the invention

The second technical problem set forth above is substantially solved by a method for analytical determination of residual aldehydes in syringes comprising the technical features set forth in one or more of the appended claims, the definitions of which form an integral part of the present description for the purpose of sufficiency of description.

Therefore, the invention relates to an analytical method for detecting residual aldehydes releasable from a syringe, the method comprising the following steps:

i) Preparation of the sample to be analyzed by isolating the cone 2 of the syringe 1 comprising the portion of needle 3 inserted therein and the glue 4;

ii) Derivatization treatment of the sample of step i) with a compound of formula Ar-NH-NH$_2$, in which Ar is a phenyl substituted by one or more electron-withdrawing groups;

iii) Analysis of the solution resulting from step ii) by reverse phase HPLC coupled to UV detector.

The present invention further relates to a method for extracting residual aldehydes from syringes comprising a step of treating said syringes at a temperature higher than or equal to 50°C, preferably higher than or equal to 90°C.

Further features and advantages of the invention will become apparent from the following description of preferred embodiments, given by way of non-limiting example.

## Brief description of the drawings

Figure 1 depicts the cone of a syringe with a coupled needle in section;

Figures 2A, 2B and 2C show graphs showing the reduction of the aldehydes releasable from syringes following the treatment according to the invention.

## Detailed description of the invention

In a first aspect, the present invention relates to a method for extracting residual aldehydes from syringes comprising a step of treating said syringes at a temperature higher than or equal to 50°C, preferably higher than or equal to 90°C.

The term "syringe" or "syringes" within the meaning of the present invention means a syringe-needle assembly, in which the needle is coupled to the cone of the syringe by means of an adhesive or glue containing residual aldehydes and in which the syringe does not comprise rubber parts. This glue is typically of the UV-radiation cross-linkable type.

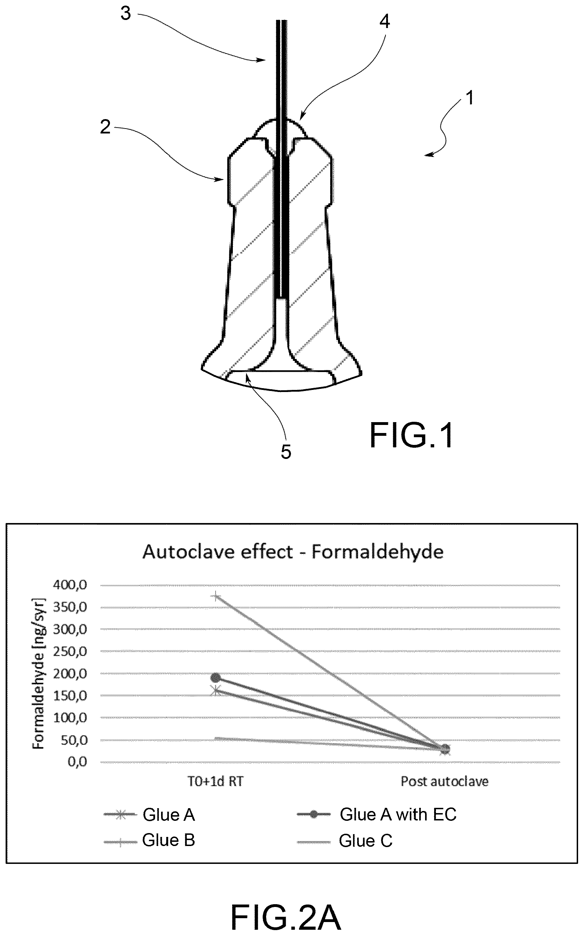

Referring to figure 1, which shows a partial view of a syringe 1, in particular only the section of the cone 2, the conventional method of coupling the needle 3 to the syringe 1 comprises the following steps:

a) preassembling the needle 3 in the cone 2,

b) dosing the glue 4 in the cone 2,

c) drawing the glue 4 from the base of the flange 5,

d) cross-linking the glue with UV lamps (LED or mercury), for example.

In certain embodiments, the pre-assembly step a) is preceded by a plasma treatment under oxygen atmosphere on the needle 3 to be glued. Such a treatment increases the wettability and surface energy of the needle 3, causing a better cleaning of the surface and therefore making it less prone to release contaminants upon gluing, possibly due to the reaction of the material of the needle with the glue.

The method for extracting residual aldehydes from syringes according to the invention comprises, as mentioned, a step of treating said syringes at a temperature higher than or equal to 50°C, preferably higher than or equal to 90°C. Such a treatment step can be carried out under vacuum, at atmospheric pressure in a liquid or gaseous fluid or under vapor pressure. Alternative methods can be:

- Autoclave under water vapor pressure;
- Ultrasonic heated bath;
- Storage at 50°C for 24 hours;
- Vacuum storage at T>50°C with continuous extraction to balance the vacuum, and prevent the vacuum bell from becoming saturated with volatile compounds;
- Washing with water at T=90°C or at boiling point.

A particularly preferred method comprises a step of treating the syringe at a temperature between 110°C and 130°C and at a water vapor pressure between 1.2 and 3 bar. More preferably, the temperature is between 118°C and 123°C and the pressure is between 1.8 and 2.3 bar.

The treatment time is an important parameter, as times of 60 minutes or more, or 90 minutes or more, do not make significant improvements, but tend to worsen the tensile strength of the needle, or even cause it to detach already in the absence of load. The treatment time is thus between 5 and 50 minutes, or between 15 and 40 minutes, or between 15 and 25 minutes, or between 20 and 35 minutes, or between 25 and 30 minutes.

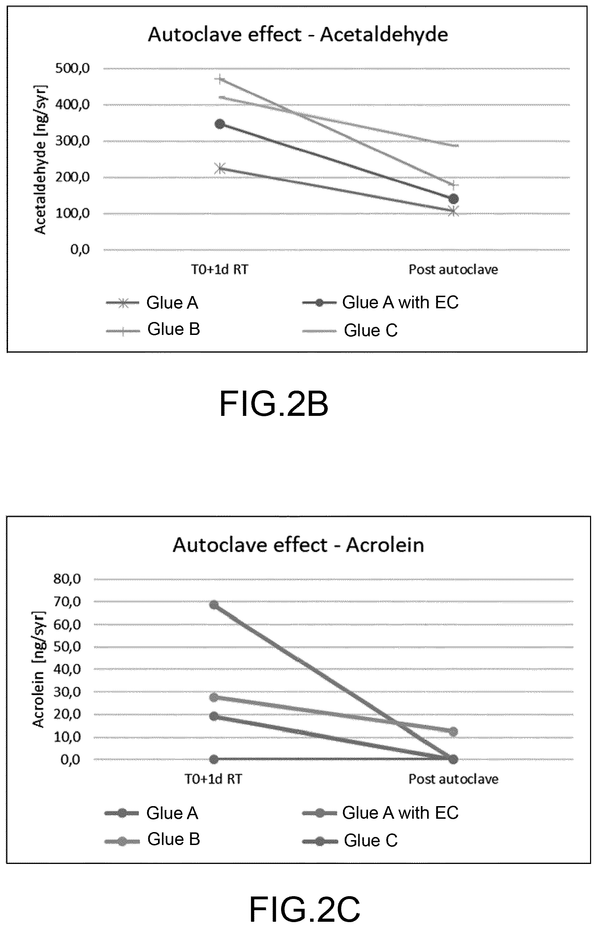

Figures 2A, 2B and 2C depict graphs showing the reduction of formaldehyde, acetaldehyde and acrolein, respectively, released from syringes in which three different glues (glue A, glue B and glue C) with different initial aldehyde contents were used. The glues used are commercial photopolymerized acrylic glues. The measurements were conducted immediately downstream of the syringe production. In all the cases, a substantial reduction in the content of aldehydes releasable from the syringe is achieved, which makes the syringes treated according to the method of the invention usable with any biological fluid, comprising a fluid containing protein material. Glue A was subjected to the autoclaving process with ("glue A with EC" in the graphs) and without *extra-curing*, i.e., with and without a process of further exposure to UV rays with respect to standard times, showing how this parameter does not affect the final result.

Therefore, a syringe obtainable by the method of the invention and having a releasable aldehyde content as follows forms a second object of the invention:

- Formaldehyde < 60 ng/syringe
- Acetaldehyde < 300 ng/syringe, preferably < 200 ng/syringe
- Acrolein < 20 ng/syringe, preferably less than 10 ng/syringe.

The evaluation of the content of aldehydes released from the syringes was carried out by a method developed by the current inventors and forming a further object of the present invention.

Such a method will be described below.

The analytical method of the invention comprises the following steps:

iv) Preparation of the sample to be analyzed by isolating the cone 2 of the syringe 1 comprising the portion of needle 3 inserted therein and the glue 4;

v) Derivatization treatment of the sample of step i) with a compound of formula $Ar-NH-NH_2$, in which Ar is a phenyl substituted by one or more electron-withdrawing groups;

vi) Analysis of the solution resulting from step ii) by reverse phase HPLC coupled to UV detector.

The sample preparation step i) comprises detaching, by means of a shear or other suitable tool, for example, the cone 2 from the cylindrical body of the syringe 1 and removing the protruding part of the needle 3. The portion of syringe 1 thus obtained, consisting of the cone 2, the portion of needle 3 inside the cone 2 and the glue 4 contained therein, further undergoes a crushing treatment of said syringe portion to give said sample.

The sample derivatization treatment step ii) comprises the steps of:

- Suspending said sample in a solution of water/acetonitrile/derivatization solution;
- Heating the suspension thus obtained to a temperature of 40-55°C, preferably about 50°C, and for a time of 1.5-2.5 hours, preferably about 2 hours.

The derivatization treatment allows transforming the aldehydes released by the glues into hydrazones by reaction with the compound of formula $Ar-NH-NH_2$. Such hydrazones are easily detectable by means of UV spectroscopy.

Preferably, said solution of water/acetonitrile/derivatization solution comprises 16-17 parts by volume of water, 1.5-2.5 parts by volume of acetonitrile, and 0.8-1.2 parts by volume of derivatization solution.

The derivatization solution comprises said compound of formula $Ar-NH-NH_2$, where Ar is a phenyl substituted by one or more electron-withdrawing groups, and preferably consists of 120-130 parts by weight of said compound of formula $Ar-NH-NH_2$ in 13-17 parts by volume of acetonitrile, 18-22 parts by volume of a strong acid, preferably 37% hydrochloric acid, and water to a total of 100 parts by volume.

It is important that the pH of the solution is between 1 and 1.2, more preferably about 1.1.

The compound of formula $Ar-NH-NH_2$ is more preferably 2,4-dinitrophenyl hydrazine.

In certain embodiments, it can be necessary or advisable to crystallize 2,4-dinitrophenyl hydrazine prior to use, for example by crystallizing 2,4-dinitrophenyl hydrazine through hot solubilization in acetonitrile and subsequent reprecipitation.

Step iii) of analyzing the solution of a sample resulting from step ii), containing hydrazones of the aldehydes released by the glue in said solution, comprises the steps of:

1) Calibrating an HPLC column, preferably a C18 column, coupled to a UV detector, by means of standard hydrazone calibration samples of said compound of formula Ar-NH-NH$_2$ with formaldehyde, acetaldehyde, acrolein, and methacrolein and using an appropriate eluent;

2) Injecting said sample solution into said calibrated HPLC column;

3) Calculating the area underneath the UV absorption peak of said sample solution;

4) Calculating the residual amount of aldehydes.

The eluent preferably consists of a varying composition mixture of eluent A (H2O/THF 71-74%/26-29% (V/V)) and eluent B (acetonitrile).

Preferably, the operating conditions of the column are as follows:

- Column temperature: 38-42°C, preferably 40°C
- Autosampler temperature: 2-8°C, preferably 5°C
- Injection volume: 100 μl
- UV detector wavelength: 367 nm
- Flow: 1 mL/min
- Eluent A/Eluent B 65/35 to 30/70 to 65/35.

The calculation of the residual amount of aldehydes is carried out by applying the following equation:

$$C_{sample} = (A_{sample} \times C_{standard} \times F \times D \times R)/A_{standard}$$

where

C = concentration
A = area
D = dilution factor = 1
F = conversion factor from derivatized aldehyde to free aldehyde
R = correction factor of the derivatization yield.

Factors R and F are shown in the following table:

| Aldehyde | Factor F | Factor R |
|---|---|---|
| Formaldehyde | 0.1415 | 1.31 |
| Acetaldehyde | 0.1965 | 2.42 |
| Acrolein | 0.2374 | 2.06 |
| Methacrolein | 0.2801 | 1.57 |

It has been shown that the choice of 2,4-dinitro-phenylhydrazine for derivatization leads to very satisfactory results.

The method thus defined has a specificity, that is, an ability to discriminate the substances sought with respect to noise, in accordance with the validation standards required by the main pharmacopoeias.

The quantification limit of the method thus exposed is highly low, and thus adapted to detect even minimal amounts of aldehydes present in the samples analyzed; the lowest quantifiable signal with respect to background noise was around 1-2 ng/ml per syringe.

The coefficient of variation (RSDx100, where RSD= relative standard deviation given by SD/mean) of the measurements according to the method of the invention is less than or equal to 3%.

The method is also stable within 10% with respect to a sample storage time of 2h at room T, or 6h at 5°C.

ALDEHYDE CONTENT REDUCTION TESTS

Table I below summarizes the tests conducted on uncapped syringes using the various aldehyde reduction methods described above. The determination of the amount of residual aldehydes was carried out as described above. In all the cases, Loctite® AA 3345, produced by Henkel, Duesseldorf was used as a glue. The test was carried out on 5 syringes for each type of treatment and the mean value of residual aldehydes $\pm$ S.D. was calculated.

(Standard Deviation).

N.T. treatment: no treatment

Treatment 1: autoclave, P=2 bar, T=121°C, t=20 minutes Treatment 2: autoclave, P=2 bar, T=121°C, t=60 minutes Treatment 3: dry vacuum heating, T=60°C, t=60 minutes Treatment 4: storage at T=50°C, P=atm, t=1 month Treatment 5: under vacuum at T=ambient, t=24 hours Treatment 6: ultrasonic bath, P=atm, T=75°C, t=3 hours. Treatment 1 was also repeated on capped syringes in which a rubber part is present (treatment 7).

Table I

| treatment | Formaldehyde ng/ syringe | Acetaldehyde ng/ syringe | Acrolein ng/ syringe | Methacrolein ng/ syringe |
|---|---|---|---|---|
| N.T. | 37.1 $\pm$ 9.1 | 1266.3 $\pm$ 884.0 | 31.1 $\pm$ 2.4 | 2.7 |
| 1 | 13.8 $\pm$ 2.7 | 236.6 $\pm$ 144.9 | 3.8 $\pm$ 0.0 | 2.9 $\pm$ 03 |
| 2 | 22.1 $\pm$ 13.8 | 395.3 $\pm$ 232.7 | - | 3.0 $\pm$ 0.4 |
| 3 | 145.0 $\pm$ 17.9 | 331.0 $\pm$ 54.0 | 63.5 $\pm$ 5.6 | - |
| 4 | 17.0 $\pm$ 4.8 | 157.8 $\pm$ 87.0 | 21.3 $\pm$ 1.0 | - |
| 5 | 170.7 $\pm$ 35.7 | 263.2 $\pm$ 14.6 | 60.6 $\pm$ 4.2 | - |
| 6 | 22.3 $\pm$ 2.7 | 127.1 $\pm$ 15.7 | 11.8 $\pm$ 0.6 | 2.8 $\pm$ 0.1 |
| 7 | 74.2 $\pm$ 2.7 | 400.2 $\pm$ 46.6 | 7.2 $\pm$ 1.0 | - |

The above data show that the 3-hour ultrasonic bath at 75° and the autoclaving at 121°C, in particular by reducing the time from 60 minutes to 20 minutes, provide the best reduction of aldehyde content.

Treatments 4 and 6, while resulting in a good reduction of formaldehyde and acetaldehyde, do not provide a sufficient reduction of acrolein (particularly critical component) when compared to methods 1 and 2 in an autoclave. Furthermore, they require too long processing times for an industrial implementation thereof.

The information related to the comparison of the data in autoclave at 121°C for 20 minutes with (treatment 1) and without (treatment 7) cap deserve particular discussion. These data indicate that the cap, which has rubber parts, significantly contributes to the release of aldehydes. The sterilization in an autoclave without the presence of rubber parts is thus improved.

It should be noted that the removal of rubber parts to prevent a possible adhesion of the cap to the needle at high temperature due to the softening of the rubber would not be necessary per se, since the materials used for the cap, including the rubber, are made to resist without "stitching" at the sterilization temperatures in the autoclave, a treatment often used by many manufacturers. However, the present inventors have verified that sterilizing in an autoclave with the cap leads to higher aldehyde contamination of the cone, since the material of the cap contains many more aldehydes, and tends to release them even in the cone, as well as in the autoclaving environment (where they are removed).

This results in a syringe which retains, even downstream of the autoclave, a higher content of aldehydes.

By autoclaving the syringe alone, the aldehydes in the syringe are then removed in a more effective manner. On the other hand, the cap, when assembled after autoclaving, does not contribute to the contamination of the syringe by aldehydes, as the contact surface between the cap and the drug is limited to the circular area related to the needle cannula and there is a blast of air between the rubber and the drug.

It is apparent that only some particular embodiments of the present invention have been described, to which those skilled in the art will be able to make all changes required to adapt it to particular applications, without departing from the scope of protection of the present invention.

## Claims

1. An analytical method for detecting residual aldehydes releasable from a syringe (1), wherein said syringe is a syringe (1)-needle (3) assembly, wherein the needle (3) is coupled to the cone (2) of the syringe (1) by means of an adhesive or glue (4) containing residual aldehydes, the method comprising the following steps:

i) preparation of the sample to be analysed by isolating the cone (2) of the syringe (1) comprising the needle portion (3) inserted in it and the glue (4) ;

ii) derivatization treatment of the sample of step i) with a compound of formula Ar-NH-NH$_2$, wherein Ar is a phenyl substituted with one or more electron-attractant groups;

iii) analysis of the solution resulting from step ii) by reverse-phase HPLC coupled to UV detector,

wherein step i) of preparing the sample comprises detaching the cone (2) from the cylindrical body of the syringe (1) and removing the protruding needle portion (3), and wherein the syringe portion (1) thus obtained, constituted by the cone (2), the needle portion (3) inside the cone (2) and the glue (4) therein, further undergoes a treatment of crushing said syringe portion to give said sample.

2. The method according to claim 1, wherein the step ii) of derivatization treatment of the sample comprises the steps of:

- suspension of said sample in a solution of water/acetonitrile/derivatization solution;
- heating of the suspension thus obtained at a temperature of 40-55°C, or about 50°C, and for a time of 1.5-2.5 hours, or about 2 hours.

3. The method according to claim 2, wherein said solution of water/acetonitrile/derivatization solution comprises 16-17 parts by volume of water, 1.5-2.5 parts by volume of acetonitrile, and 0.8-1.2 parts by volume of derivatization solution.

4. The method according to claim 2 or 3, wherein said derivatization solution comprises said compound of formula Ar-NH-NH$_2$, wherein Ar is a phenyl substituted with one or more electron attractant groups, and is formed by 120-130 parts by weight of said compound of formula Ar-NH-NH$_2$ in 13-17 parts by volume of acetonitrile, 18-22 parts by volume of a strong acid, preferably 37% hydrochloric acid, so as to obtain a pH of between 1 and 1.2, and water up to a total of 100 parts by volume.

5. The method according to any of the claims from 1 to 4, wherein the compound of formula Ar-NH-NH$_2$ is 2,4-dinitrophenyl hydrazine.

6. The method according to any of the claims from 1 to 5, wherein step iii) of analysing the solution of a sample resulting from step ii), containing hydrazones of the aldehydes released from the glue in said solution, comprises the steps of:

1) calibrating a HPLC column, preferably a C18 column, coupled to a UV detector, using standard calibration samples of hydrazones of said compound of formula Ar-NH-NH$_2$ with formaldehyde, acetaldehyde, acrolein and methacrolein and using an appropriate eluent;
2) injecting said sample solution into said calibrated HPLC column;
3) calculating the area subtended by the UV absorption peak of said sample solution;
4) calculating the residual quantity of aldehydes.

7. The method according to claim 6, wherein the eluent consists of a mixture having a variable composition of eluent A comprising H2O/THF 71-74%/26-29% (V/V)) and eluent B being acetonitrile.

8. The method according to claim 7, wherein the operating values of the columns are as follows:

- Column temperature: 38-42°C, preferably 40°C
- Autosampler temperature: 2-8°C, preferably 5°C
- Injection volume: 100 μl
- UV detector wavelength: 367 nm
- Flow: 1 mL/min
- Eluent A/Eluent B from 65/35 to 30/70 to 65/35.

9. The method according to any of the claims from 6 to 8, wherein the calculation of the residual quantity of aldehydes is conducted by applying the following equation:

$$C_{sample} = (A_{sample} \times C_{standard} \times F \times D \times R)/A_{standard}$$

where

C = concentration
A = Area
D = dilution factor = 1
F = conversion factor from derivatized aldehyde to free aldehyde
R = Derivatization yield correction factor, and where the factors R and F are given in the following table:

| Aldehyde | F factor | R factor |
|---|---|---|
| Formaldehyde | 0.1415 | 1.31 |
| Acetaldehyde | 0.1965 | 2.42 |
| Acrolein | 0.2374 | 2.06 |
| Methacrolein | 0.2801 | 1.57 |

**Patentansprüche**

1. Analytisches Verfahren zum Nachweis von Restaldehyden, die aus einer Spritze (1) freigesetzt werden können, wobei die Spritze eine Spritzen (1)-Nadel (3)-Anordnung ist, wobei die Nadel (3) mit dem Konus (2) der Spritze (1) mittels eines Haft- oder Klebstoffs (4) gekoppelt ist, der Restaldehyde enthält, wobei das Verfahren die folgenden Schritte umfasst:

   i) Vorbereitung bzw. Herstellung der zu analysierenden Probe durch Isolieren des Konus (2) der Spritze (1), der den darin eingesetzten Nadelabschnitt (3) und den Klebstoff (4) umfasst;
   ii) Derivatisierungsbehandlung der Probe aus Schritt i) mit einer Verbindung der Formel Ar-NH-NH$_2$, worin Ar ein mit einer oder mehreren elektronenanziehenden Gruppen substituiertes Phenyl ist;
   iii) Analyse der aus Schritt ii) resultierenden Lösung durch Umkehrphasen-HPLC, gekoppelt mit einem UV-Detektor,

   wobei Schritt i) der Herstellung der Probe ein Ablösen des Konus (2) von dem zylindrischen Körper der Spritze (1) und ein Entfernen des vorstehenden Nadelabschnitts (3) umfasst und wobei der so erhaltene Spritzenabschnitt (1), der aus dem Konus (2), dem Nadelabschnitt (3) innerhalb des Konus (2) und dem Klebstoff (4) darin gebildet ist, ferner einer Behandlung des Zerkleinerns des Spritzenabschnitts unterzogen wird, um die Probe zu erhalten.

2. Verfahren nach Anspruch 1, wobei der Schritt ii) der Derivatisierungsbehandlung der Probe die Schritte umfasst:

   - Suspendieren der Probe in einer Lösung aus Wasser/Acetonitril/Derivatisierungslösung;
   - Erhitzen der so erhaltenen Suspension auf eine Temperatur von 40-55°C oder etwa 50°C und für eine Dauer von 1,5-2,5 Stunden oder etwa 2 Stunden.

3. Verfahren nach Anspruch 2, wobei die Lösung aus Wasser/Acetonitril/Derivatisierungslösung 16-17 Volumenteile Wasser, 1,5-2,5 Volumenteile Acetonitril und 0,8-1,2 Volumenteile Derivatisierungslösung umfasst.

4. Verfahren nach Anspruch 2 oder 3, wobei die Derivatisierungslösung die Verbindung der Formel Ar-NH-NH$_2$ umfasst, wobei Ar ein mit einer oder mehreren elektronenanziehenden Gruppen substituiertes Phenyl ist, und aus 120-130 Gewichtsteilen der Verbindung der Formel Ar-NH-NH$_2$ in 13-17 Volumenteilen Acetonitril, 18-22 Volumenteilen einer starken Säure, vorzugsweise 37%iger Salzsäure, um einen pH-Wert zwischen 1 und 1,2 zu erhalten, und Wasser bis zu einer Gesamtmenge von 100 Volumenteilen gebildet wird.

5. Verfahren nach einem der Ansprüche 1 bis 4, wobei die Verbindung der Formel Ar-NH-NH$_2$ 2,4-Dinitrophenylhydrazin ist.

6. Verfahren nach einem der Ansprüche 1 bis 5, wobei Schritt iii) der Analyse der Lösung einer aus Schritt ii) resultierenden Probe, die Hydrazone der aus dem Klebstoff freigesetzten Aldehyde in der Lösung enthält, die Schritte umfasst:

1) Kalibrieren einer HPLC-Säule, vorzugsweise einer C18-Säule, die mit einem UV-Detektor gekoppelt ist, unter Verwendung von Standardkalibrierungsproben von Hydrazonen der Verbindung der Formel Ar-NH-NH$_2$ mit Formaldehyd, Acetaldehyd, Acrolein und Methacrolein und unter Verwendung eines geeigneten Elutionsmittels;
2) Injizieren der Probenlösung in die kalibrierte HPLC-Säule;
3) Berechnen der Fläche, die durch den UV-Absorptionspeak der Probenlösung begrenzt wird;
4) Berechnen der Restmenge von Aldehyden.

7. Verfahren nach Anspruch 6, wobei das Elutionsmittel aus einem Gemisch mit einer variablen Zusammensetzung von Elutionsmittel A, das H2O/THF 71-74%/26-29% (V/V) umfasst, und Elutionsmittel B, das Acetonitril ist, besteht.

8. Verfahren nach Anspruch 7, wobei die Betriebswerte der Säulen wie folgt sind:

- Säulentemperatur: 38-42°C, vorzugsweise 40°C
- Autosampler-Temperatur: 2-8°C, vorzugsweise 5°C
- Injektionsvolumen: 100 μl
- UV-Detektor-Wellenlänge: 367 nm
- Durchfluss: 1 mL/min
- Elutionsmittel A/Elutionsmittel B von 65/35 bis 30/70 bis 65/35.

9. Verfahren nach einem der Ansprüche 6 bis 8, wobei die Berechnung der Restmenge von Aldehyden durch Anwenden der folgenden Gleichung durchgeführt wird:

$$C_{Probe} = (A_{Probe} \times C_{Standard} \times F \times D \times R) / A_{Standard}$$

wobei

C = Konzentration
A = Fläche
D = Verdünnungsfaktor = 1
F = Umrechnungsfaktor vom derivatisierten Aldehyd zum freien Aldehyd
R = Korrekturfaktor für die Derivatisierungsausbeute, wobei die Faktoren R und F in der folgenden Tabelle gegeben sind:

| Aldehyd | Faktor F | Faktor R |
|---|---|---|
| Formaldehyd | 0,1415 | 1,31 |
| Acetaldehyd | 0,1965 | 2,42 |
| Acrolein | 0,2374 | 2,06 |
| Methacrolein | 0,2801 | 1,57 |

**Revendications**

1. Procédé d'analyse pour détecter des aldéhydes résiduels pouvant se libérer d'une seringue (1), dans lequel ladite seringue est un ensemble seringue (1)-aiguille (3), dans lequel l'aiguille (3) est couplée au cône (2) de la seringue (1) au moyen d'un adhésif ou d'une colle (4) contenant des aldéhydes résiduels, le procédé comprenant les étapes suivantes :

i) la préparation de l'échantillon à analyser par isolement du cône (2) de la seringue (1) comprenant la partie aiguille (3) insérée dans celui-ci et la colle (4) ;
ii) le traitement de dérivatisation de l'échantillon de l'étape i) avec un composé de formule Ar-NH-NH$_2$, dans lequel Ar est un phényle substitué par un ou plusieurs groupes attracteurs d'électrons ;
iii) l'analyse de la solution résultant de l'étape ii) par HPLC en phase inverse couplée à un détecteur UV,

dans lequel l'étape i) de préparation de l'échantillon comprend la séparation du cône (2) vis-à-vis du corps cylindrique de la seringue (1) et le retrait de la partie d'aiguille en saillie (3), et dans lequel la partie de seringue (1) ainsi obtenue, constituée du cône (2), de la partie d'aiguille (3) à l'intérieur du cône (2) et de la colle (4) à l'intérieur de celui-ci, subit en outre un traitement de broyage de ladite partie de seringue pour donner ledit échantillon.

2. Procédé selon la revendication 1, dans lequel l'étape ii) de traitement de dérivatisation de l'échantillon comprend les étapes suivantes :

- mettre en suspension ledit échantillon dans une solution d'eau/d'acétonitrile/de solution de dérivatisation ;
- chauffer la suspension ainsi obtenue à une température de 40 à 55 °C, ou d'environ 50 °C, et pendant une durée de 1,5 à 2,5 heures, ou d'environ 2 heures.

3. Procédé selon la revendication 2, dans lequel ladite solution d'eau/d'acétonitrile/de solution de dérivatisation comprend 16 à 17 parties en volume d'eau, 1,5 à 2,5 parties en volume d'acétonitrile, et 0,8 à 1,2 partie en volume de solution de dérivatisation.

4. Procédé selon la revendication 2 ou 3, dans lequel ladite solution de dérivatisation comprend ledit composé de formule $Ar-NH-NH_2$, dans lequel Ar est un phényle substitué par un ou plusieurs groupes attracteurs d'électrons, et est formée de 120 à 130 parties en poids dudit composé de formule $Ar-NH-NH_2$ dans 13 à 17 parties en volume d'acétonitrile, 18 à 22 parties en volume d'un acide fort, de préférence de l'acide chlorhydrique à 37 %, de manière à obtenir un pH compris entre 1 et 1,2, et de l'eau jusqu'à un total de 100 parties en volume.

5. Procédé selon l'une quelconque des revendications 1 à 4, dans lequel le composé de formule $Ar-NH-NH_2$ est de la 2,4-dinitrophénylhydrazine.

6. Procédé selon l'une quelconque des revendications 1 à 5, dans lequel l'étape iii) d'analyse de la solution d'un échantillon résultant de l'étape ii), contenant des hydrazones des aldéhydes libérés de la colle dans ladite solution, comprend les étapes suivantes :

1) étalonner une colonne HPLC, de préférence une colonne C18, couplée à un détecteur UV, en utilisant des échantillons d'étalonnage standard d'hydrazones dudit composé de formule $Ar-NH-NH_2$ avec du formaldéhyde, acétaldéhyde, acroléine et méthacroléine et en utilisant un éluant approprié ;
2) injecter ladite solution échantillon dans ladite colonne HPLC étalonnée ;
3) calculer la surface sous-tendue par le pic d'absorption UV de ladite solution échantillon ;
4) calculer la quantité résiduelle d'aldéhydes.

7. Procédé selon la revendication 6, dans lequel l'éluant est constitué d'un mélange ayant une composition variable d'éluant A comprenant H2O/THF 71-74 %/26-29 % (V/V) et d'éluant B qui est de l'acétonitrile.

8. Procédé selon la revendication 7, dans lequel les valeurs de fonctionnement des colonnes sont les suivantes :

- température de colonne : 38-42 °C, de préférence 40 °C
- température d'auto-échantillonneur : 2-8 °C, de préférence 5 °C
- volume d'injection : 100 $\mu$l
- longueur d'onde détecteur UV : 367 nm
- Débit : 1 ml/min
- Éluant A/Éluant B de 65/35 à 30/70 à 65/35.

9. Procédé selon l'une quelconque des revendications 6 à 8, dans lequel le calcul de la quantité résiduelle d'aldéhydes est effectué en appliquant l'équation suivante :

$$C_{\text{échantillon}} = (A_{\text{échantillon}} \times C_{\text{standard}} \times F \times D \times R) / A_{\text{standard}}$$

où

C = concentration
A = surface

D = facteur de dilution = 1

F = facteur de conversion d'aldéhyde dérivatisé en aldéhyde libre

R = facteur de correction de rendement de dérivatisation, et où les facteurs R et F sont donnés dans le tableau suivant :

| Aldéhyde | facteur F | facteur R |
|---|---|---|
| Formaldéhyde | 0,1415 | 1,31 |
| Acétaldéhyde | 0,1965 | 2,42 |
| Acroléine | 0,2374 | 2,06 |
| Méthacroléine | 0,2801 | 1,57 |

FIG.1

FIG.2A

FIG.2B

FIG.2C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102411034 A
- US 20160251261 A1
- EP 3496765 A2

**Non-patent literature cited in the description**

- **DENFENG LIU et al.** Interactions between Therapeutic Proteins and Acrylic Acid Leachable. *PDA J. Pharm. Sci. And Tech.,* 2012, vol. 66, 12-19